(19) 

Europäisches
Patentamt
European
Patent Office
Office européen
des brevets

(11) **EP 4 717 304 A1**

(12) **EUROPEAN PATENT APPLICATION**
published in accordance with Art. 153(4) EPC

(43) Date of publication:
**01.04.2026 Bulletin 2026/14**

(21) Application number: **24814284.6**

(22) Date of filing: **23.05.2024**

(51) International Patent Classification (IPC):
***A61N 5/10*** (2006.01)

(52) Cooperative Patent Classification (CPC):
**A61N 5/10; G16C 20/10**

(86) International application number:
**PCT/CN2024/094828**

(87) International publication number:
**WO 2024/245085 (05.12.2024 Gazette 2024/49)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **01.06.2023 CN 202310644066**

(71) Applicant: **Neuboron Therapy System Ltd.
Xiamen, Fujian 361026 (CN)**

(72) Inventors:
• **ZHONG, Wan-bing**
  **Nanjing, Jiangsu 211112 (CN)**
• **CHEN, Jiang**
  **Nanjing, Jiangsu 211112 (CN)**

(74) Representative: **Gunzelmann, Rainer
Wuesthoff & Wuesthoff
Patentanwälte und Rechtsanwalt PartG mbB
Schweigerstraße 2
81541 München (DE)**

(54) **DOSE EVALUATION METHOD, IRRADIATION CONDITION SELECTION METHOD AND TREATMENT PLAN GENERATION APPARATUS**

(57)  The present application relates to a dose evaluation method, an irradiation condition selection method and a treatment plan generation apparatus. The dose evaluation method includes: acquiring information of a tissue material; determining a conjugate flux corresponding to the tissue material from the information of the tissue material; and based on an irradiation condition and in combination with the conjugate flux corresponding to the tissue material, obtaining an absorbed radiation dose corresponding to the tissue material under the irradiation condition. By using the method, an absorbed radiation dose corresponding to a tissue material can be quickly calculated, which improves the efficiency of dose calculation under a plurality of irradiation conditions, and enables the quick evaluation of the absorbed radiation doses corresponding to each tissue material under different irradiation conditions, so as to facilitate the quick selection of a suitable irradiation condition from the plurality of irradiation conditions, thereby greatly shortening the formulation time of a treatment plan.

EP 4 717 304 A1

```
┌─────────────────────────────────────────────┐
│      Acquire information of a tissue material │
└─────────────────────────────────────────────┘
                      │
                      ▼
┌─────────────────────────────────────────────┐
│  Determine a conjugate flux corresponding to  │
│  the tissue material from the information of   │
│  the tissue material                           │
└─────────────────────────────────────────────┘
                      │
                      ▼
┌─────────────────────────────────────────────┐
│  Based on an irradiation condition and in      │
│  combination with the conjugate flux           │
│  corresponding to the tissue material, obtain  │
│  an absorbed radiation dose corresponding to   │
│  the tissue material under the irradiation     │
│  condition                                     │
└─────────────────────────────────────────────┘
```

*FIG. 2*

## Description

### TECHNICAL FIELD

The present application relates to the technical field of radiation therapies, and in particular, to a dose evaluation method, an irradiation condition selection method, and a treatment plan generation apparatus.

### BACKGROUND

Radiation therapy is a means of medical treatment of cancer by using radiation to deposit energy around cancer cells to kill cancer cells. Depending on the types of radiation, radiation therapy can be classified into photon radiation therapy, electron radiation therapy, proton and heavy ion radiation therapy, boron neutron capture therapy, etc.

To maximize the killing of cancer cells by radiation particles while minimizing damage to normal cells, imaging scans such as Computed Tomography (CT) or Positron Emission Computed Tomography (PET) are typically performed on a patient prior to treatment. Based on scan results, information of a tissue material of an irradiated body is obtained. A computational model is then established based on the information of the tissue material and a radiation source to simulate the transport process of radiation particles within the irradiated body. Finally, the dose distribution of the radiation particles in the irradiated body is obtained, and a treatment plan with an optimal dose distribution for the patient is selected as a treatment scheme for the patient.

During the generation of the foregoing treatment plan, the calculation of the dose distribution in an irradiated site of the patient is crucial to the efficacy of radiation therapy. The calculation of the dose distribution is usually performed under a given irradiation condition, and the selection of the irradiation condition often depends on a physicist's own experience, which has a great uncertainty. In traditional technologies, the dose distribution is mainly obtained by simulating the movement process of radiation particles using the Monte Carlo method, in which the transport process of radiation particles in the irradiated body under different irradiation conditions is simulated to obtain the dose distributions in the patient under different irradiation conditions. It is necessary to simulate the movement process of photons and electrons for traditional radiation therapies, and to simulate the movement process of neutrons and photons for other radiation therapies.

However, the simulation and calculation processes of the aforementioned method are time-consuming and consume large amounts of memory. Moreover, there are many pre-given irradiation conditions, and the simulation and calculation for the given irradiation conditions one by one using the aforementioned method takes a long time. Therefore, it is difficult to select a suitable irradiation condition within a reasonable calculation time, which is not conducive to the optimal calculation of a treatment plan.

### SUMMARY

Based on this, it is necessary to provide a dose evaluation method, an irradiation condition selection method and a treatment plan generation apparatus that enable the quick evaluation of a dose and the quick selection of a suitable irradiation condition for the above technical problems.

In a first aspect, the present application provides a radiation therapy dose evaluation method. The method includes:

acquiring information of a tissue material;
determining a conjugate flux corresponding to the tissue material from the information of the tissue material; and
based on an irradiation condition and in combination with the conjugate flux corresponding to the tissue material, obtaining an absorbed radiation dose corresponding to the tissue material under the irradiation condition.

In one embodiment, determining the conjugate flux corresponding to the tissue material from the information of the tissue material includes:

determining a flux-dose conversion factor corresponding to the tissue material from element information of the tissue material in the information of the tissue material; and
determining the conjugate flux corresponding to the tissue material from the flux-dose conversion factor corresponding to the tissue material.

In one embodiment, the element information includes an element composition and an element proportion, and determining the flux-dose conversion factor corresponding to the tissue material from the element information of the tissue material in the information of the tissue material includes:

acquiring a density of each nuclide in the tissue material from the element composition and the element proportion;
acquiring a flux-dose conversion factor corresponding to each nuclide; and
determining the flux-dose conversion factor corresponding to the tissue material from the flux-dose conversion factor corresponding to each nuclide and the density of each nuclide in combination.

In one embodiment, the flux-dose conversion factor corresponding to the tissue material is determined from the flux-dose conversion factor corresponding to each nuclide and the density of each nuclide in combination by

$$Kerma = \sum \rho_i \cdot Kerma_i;$$

where $\rho_i$ represents a density of an i-th nuclide, and *Kerma$_i$* represents a flux-dose conversion factor corresponding to the i-th nuclide.

In one embodiment, the conjugate flux corresponding to the tissue material is determined from the flux-dose conversion factor corresponding to the tissue material by

$$\emptyset^*(P_1) = Kerma(P_1) + \int K^*(P_2 \to P_1)\emptyset^*(P_2)dP_2;$$

where $\emptyset^*$ represents a conjugate flux, $P_1$ and $P_2$ represent different particle state parameters, $\emptyset^*(P_1)$ represents a conjugate flux of particles in the state $P_1$, *Kerma* represents a flux-dose conversion factor, $K^*$ represents a conjugate kernel, and $K^*(P_2 \to P_1)$ represents a probability that a particle transitions from the state $P_1$ to the state $P_2$ by a collision reaction.

In one embodiment, based on the irradiation condition and in combination with the conjugate flux corresponding to the tissue material, obtaining the absorbed radiation dose corresponding to the tissue material under the irradiation condition include:

determining a source term distribution corresponding to the irradiation condition based on the irradiation condition, where the source term distribution is used to characterize a distribution function of a particle source under the irradiation condition; and
acquiring the absorbed radiation dose corresponding to the tissue material from the source term distribution and the conjugate flux corresponding to the tissue material.

In a second aspect, the present application further provides an irradiation condition selection method. The method includes:

obtaining an absorbed radiation dose corresponding to each tissue material from an irradiation condition and a conjugate flux corresponding to the tissue material in combination; and
selecting at least one suitable irradiation condition based on the absorbed radiation dose corresponding to each tissue material.

In one embodiment, selecting at least one suitable irradiation condition based on the absorbed radiation dose corresponding to each tissue material includes:
accepting the irradiation condition if the absorbed radiation dose corresponding to each tissue material meets a preset condition.

In one embodiment, the tissue material includes at least a tumor tissue material and an important organ tissue material, and accepting the irradiation condition if the absorbed radiation dose corresponding to each tissue material meets the preset condition includes:
if the absorbed radiation dose corresponding to the tumor tissue material is greater than a first preset threshold, and the absorbed radiation dose corresponding to the important organ tissue material is less than a second preset threshold, accepting the irradiation condition.

In one embodiment, the method includes, prior to obtaining the absorbed radiation dose corresponding to each tissue material from the irradiation condition and the conjugate flux corresponding to the tissue material in combination, based on at least one preset region of interest, determining a tissue material corresponding to the region of interest.

In one embodiment, the method includes, prior to obtaining the absorbed radiation dose corresponding to each tissue material from the irradiation condition and the conjugate flux corresponding to the tissue material in combination, based on at least one preset region of interest and in combination with a concentration distribution of a target element in the region of interest, determining a tissue material corresponding to the region of interest.

In one embodiment, selecting the suitable irradiation condition based on the absorbed radiation dose corresponding to each tissue material includes:

determining an absorbed radiation dose for the region of interest from absorbed radiation doses corresponding to all tissue materials in the region of interest; and
selecting the suitable irradiation condition based on the absorbed radiation dose for the region of interest.

In a third aspect, the present application further provides a treatment plan generation method. The method includes:

obtaining an absorbed radiation dose corresponding to each tissue material from an irradiation condition and a conjugate flux corresponding to the tissue material in combination; and
selecting at least one suitable irradiation condition based on the absorbed radiation dose corresponding to each tissue material; and
generating a treatment plan based on the suitable irradiation condition.

In one embodiment, selecting at least one suitable irradiation condition based on the absorbed radiation dose corresponding to each tissue material includes:
accepting the irradiation condition if the absorbed radiation dose corresponding to each tissue material meets a preset condition.

In one embodiment, the tissue material includes at least a tumor tissue material and an important organ tissue material, and accepting the irradiation condition if the absorbed radiation dose corresponding to each tissue material meets the preset condition includes:
if the absorbed radiation dose corresponding to the tumor tissue material is greater than a first preset threshold, and the absorbed radiation dose corresponding to the important organ tissue material is less than a second preset threshold, accepting the irradiation condition.

In one embodiment, the method includes, prior to obtaining the absorbed radiation dose corresponding to each tissue material from the irradiation condition and the conjugate flux corresponding to the tissue material in combination, based on at least one preset region of interest, determining a tissue material corresponding to the region of interest.

In one embodiment, the method includes, prior to obtaining the absorbed radiation dose corresponding to each tissue material from the irradiation condition and the conjugate flux corresponding to the tissue material in combination, based on at least one preset region of interest and in combination with a concentration distribution of a target element in the region of interest, determining a tissue material corresponding to the region of interest.

In one embodiment, selecting the suitable irradiation condition based on the absorbed radiation dose corresponding to each tissue material includes:

determining an absorbed radiation dose for the region of interest from absorbed radiation doses corresponding to all tissue materials in the region of interest; and
selecting the suitable irradiation condition based on the absorbed radiation dose for the region of interest.

In one embodiment, generating the treatment plan based on the suitable irradiation condition includes:
determining a corresponding flux based on the suitable irradiation condition, and performing dose distribution calculation using a Monte Carlo simulation program based on the flux.

In a fourth aspect, the present application further provides a treatment plan generation apparatus. The treatment plan generation apparatus includes:

a radiation source module configured to establish source term information based on different irradiation conditions;
a medical imaging data module configured to acquire information of a tissue material based on medical imaging data and establish a three-dimensional voxel tissue model;
a dose evaluation module configured to determine a conjugate flux corresponding to the tissue material from the information of the tissue material, and obtain an absorbed radiation dose corresponding to each tissue material from the source term information and the conjugate flux corresponding to the tissue material in combination;
an irradiation condition selection module configured to select at least one suitable irradiation condition based on the absorbed radiation dose corresponding to each tissue material; and
a treatment plan generation module configured to generate a treatment plan based on the suitable irradiation condition.

In a fifth aspect, the present application further provides a radiation therapy system. The system includes:

a beam generation apparatus configured to generate a therapeutic beam;

a beam adjustment apparatus configured to adjust the therapeutic beam generated by the beam generation apparatus;

a treatment plan generation apparatus configured to establish source term information based on different irradiation conditions, acquire information of a tissue material based on medical imaging data, establish a three-dimensional voxel tissue model, determine a conjugate flux corresponding to the tissue material from the information of the tissue material, obtain an absorbed radiation dose corresponding to each tissue material from the source term information and the conjugate flux corresponding to the tissue material in combination, select at least one suitable irradiation condition based on the absorbed radiation dose corresponding to each tissue material, and generate a treatment plan based on the suitable irradiation condition; and

a beam control apparatus configured to retrieve a corresponding treatment plan from the treatment plan generation apparatus and control the beam generation apparatus to perform the treatment plan.

In a sixth aspect, the present application further provides a radiation therapy system. The system includes a memory and a processor.

The memory is configured to store a computer program.

The processor is configured to, when executing the computer program, cause the radiation therapy dose evaluation method provided in the first aspect of the present application, the irradiation condition selection method provided in the second aspect of the present application, or the treatment plan generation method provided in the third aspect of the present application to be implemented.

In a seventh aspect, the present application further provides a computer-readable storage medium, on which a computer program is stored, where the computer program, when executed by a processor, causes the radiation therapy dose evaluation method provided in the first aspect of the present application, the irradiation condition selection method provided in the second aspect of the present application, or the treatment plan generation method provided in the third aspect of the present application to be implemented.

In the radiation therapy dose evaluation method described above, the information of the tissue material is acquired, the conjugate flux corresponding to the tissue material is determined from the information of the tissue material, and based on the irradiation condition and in combination with the conjugate flux corresponding to the tissue material, the absorbed radiation dose corresponding to the tissue material under the irradiation condition is obtained. Since the evaluation of the absorbed radiation dose from the irradiation condition and the conjugate flux in combination is relatively simple, the use of the method described above enables quick evaluation of the absorbed radiation dose corresponding to the tissue material.

Further, since the conjugate flux is irrelevant to the source term S, the number of calculations for the conjugate flux is related to only the count target of tissue materials that need to be considered. Regardless of changes in irradiation conditions, the conjugate flux corresponding to a tissue material remains fixed, making it possible to quickly obtain the absorbed radiation doses corresponding to the tissue material under a plurality of different irradiation conditions, and also the absorbed radiation doses for each region of interest under a plurality of different irradiation conditions. Therefore, in the irradiation condition selection method described above, when at least one suitable irradiation condition needs to be selected from a plurality of different irradiation conditions, it is only necessary to calculate the conjugate flux several times to evaluate the absorbed radiation doses for each tissue material or each region of interest under multiple different irradiation conditions, and a suitable irradiation condition can be quickly selected based on the absorbed radiation doses under a plurality of different irradiation conditions.

Moreover, in the treatment plan generation method described above, since the suitable irradiation condition can be quickly selected from a plurality of different irradiation conditions, the formulation time of a treatment plan is greatly shortened.

## BRIEF DESCRIPTION OF THE DRAWINGS

FIG. 1 is an application environment diagram according to an embodiment;

FIG. 2 is a schematic flowchart of a radiation therapy dose evaluation method according to an embodiment;

FIG. 3 is a schematic flowchart of an irradiation condition selection method according to an embodiment;

FIG. 4 is a schematic flowchart of a treatment plan generation method according to an embodiment;

FIG. 5 is structural block diagram of a treatment plan generation apparatus according to an embodiment;

FIG. 6 is a structural block diagram of a radiation therapy system according to an embodiment; and

FIG. 7 is a structural block diagram of a radiation therapy system according to another embodiment.

# EP 4 717 304 A1

## DETAILED DESCRIPTION OF THE EMBODIMENTS

In order to make the objective, technical solutions, and advantages of the present application clearer, the present application is further described in detail below with reference to the accompanying drawings and the embodiments. It should be understood that the specific embodiments described herein are merely used to explain the present application and are not intended to limit the present application.

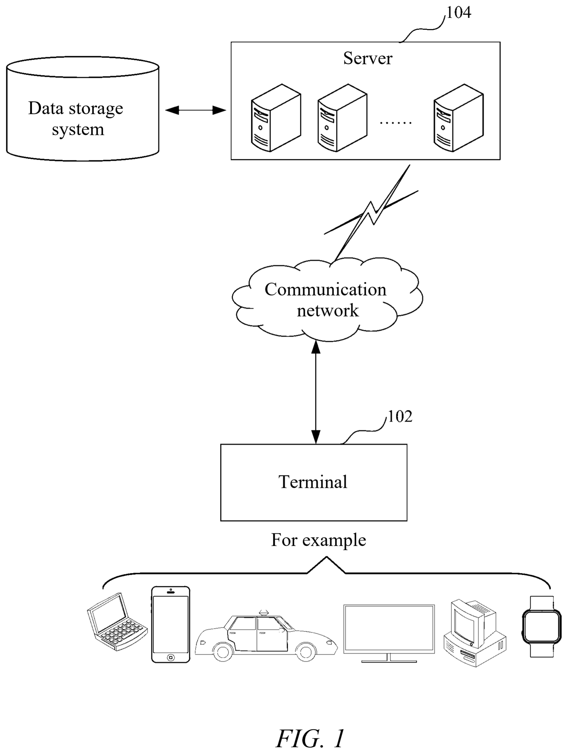

A radiation therapy dose evaluation method, an irradiation condition selection method, and a treatment plan generation method according to embodiments of the present application can be applied in application environment as shown in FIG. 1. A terminal 102 communicates with a server 104 over a network. A data storage system can store data to be processed by the server 104, and the data storage system can be integrated in the server 104, or placed on the cloud or other network servers. The terminal 102 may be, but not limited to, various personal computers, notebook computers, smart phones, tablet computers, Internet of Things devices, and portable wearable devices. The Internet of Things devices may include smart speakers, smart TVs, smart air conditioners, smart in-vehicle devices, etc. The portable wearable devices may include smart watches, smart bracelets, head-mounted devices, etc. The server 104 may be implemented with a standalone server or a cluster of servers composed of a plurality of servers.

It should be noted that the radiation therapy dose evaluation method, the irradiation condition selection method, and the treatment plan generation method according to the embodiments of the present application may generally be executed by the server 104. Accordingly, the treatment plan generation apparatus according to the embodiments of the present application may generally be disposed in the server 104. The radiation therapy dose evaluation method, the irradiation condition selection method, and the treatment plan generation method according to the embodiments of the present application may also be executed by a server or a cluster of servers that is different from the server 104 and that can communicate with the terminal 102 or the server 104. Accordingly, the treatment plan generation apparatus according to the embodiments of the present application may also be disposed in a server or a cluster of servers that is different from the server 104 and that can communicate with the terminal 102 or the server 104.

It should be understood that the numbers of terminal devices, networks and servers in FIG. 1 are merely illustrative. According to implementation requirements, there may be any numbers of terminal devices, networks and servers.

The basic physical principle of radiation therapy technology is to irradiate a tumor tissue with radiation and deposit the energy it carries at cancer cells to kill them by the interaction between the radiation and the tumor tissue. The fundamental goal is to maximize the killing of cancer cells (delivering the highest possible absorbed radiation dose to the tumor tissue) on the premise of protecting healthy tissues (delivering the lowest possible absorbed radiation dose to the healthy tissues).

The radiation therapy dose evaluation method, the irradiation condition selection method, and the treatment plan generation method according to the embodiments of the present application can be applied to radiation therapy using radiation for treatment, such as traditional radiation therapy, proton radiation therapy, X-ray radiation therapy, electron beam radiation therapy, neutron capture therapy, and boron neutron capture therapy.

In radiation therapy technology, the Monte Carlo method enables precise simulation of the trajectories and energy distribution of nucleus-particle collisions in the three-dimensional space inside an irradiated body. During simulation, to determine the absorbed radiation dose distribution in the irradiated body under a certain irradiation condition, in the traditional methods, computer technology is used to process medical imaging data, a precise grid model required by a Monte Carlo software is established, and dose simulation calculation is then performed using the Monte Carlo software. In a Monte Carlo dose calculation program, a dose $D$ is calculated using Formula 1:

$$D = <\emptyset, \ kerma> = \int \emptyset(P)kerma(P)dP; \text{(Formula 1)}$$

where $D$ is the dose, $\emptyset$ is a flux, *kerma* is a flux-dose conversion factor, $P$ is a parameter characterizing a particle state, where parameters of the particle state include spatial position $r$, angle $\Omega$, and energy $E$.

The flux $\emptyset$ is calculated using Formula 2:

$$\emptyset(P_1) = S(P_1) + \int K(P_2 \to P_1)\emptyset(P_2)dP_2; \text{(Formula 2)}$$

where, $P_1$ and $P_2$ represent different particle state parameters, $S$ represents a source term, $S(P_1)$ represents the number of particles in the state $P_1$ under an initial source state, K is a transport kernel parameter in a nuclear reaction database, and $K(P_2 \to P_1)$ represents the collision probability of particles from the state $P_2$ to the state $P_1$.

According to Formula 2, the flux $\emptyset(P_1)$ of particles in the state $P_1$ includes two parts, the first part being the number of particles in the state $P_1$ under the initial source state, and the second part being the number of particles reaching the state $P_1$ from other states (different from the state $P_1$) by collision reactions. Formula 2 integrates over all $P_2$, that is, sums the number of particles converted into $P_1$ from all states different from the state $P_1$.

In the aforementioned dose simulation calculation using the Monte Carlo method, the calculation of the flux Ø is relatively complex and time-consuming, resulting in a low dose calculation efficiency. Moreover, in radiation therapy technology, it is often necessary to calculate the dose distributions in a tissue material under numerous different irradiation conditions, so as to select a suitable irradiation condition based on the dose distributions in the tissue material under different irradiation conditions. Essentially, different irradiation conditions correspond to different source terms *S*. As can be seen from the aforementioned Formula 1 and Formula 2, the flux Ø is related to the source term S, that is, related to the irradiation conditions. When calculating the doses *D* for the tissue material under different irradiation conditions via the fluxes Ø, for each irradiation condition, it is necessary to calculate the flux Ø corresponding to the irradiation condition. However, the calculation of the flux Ø is complex and time-consuming. Therefore, when calculating the dose using the flux Ø, calculating the dose distributions in the tissue material under a plurality of irradiation conditions is often extremely time-consuming, making it difficult to select a suitable irradiation condition within a short period of time.

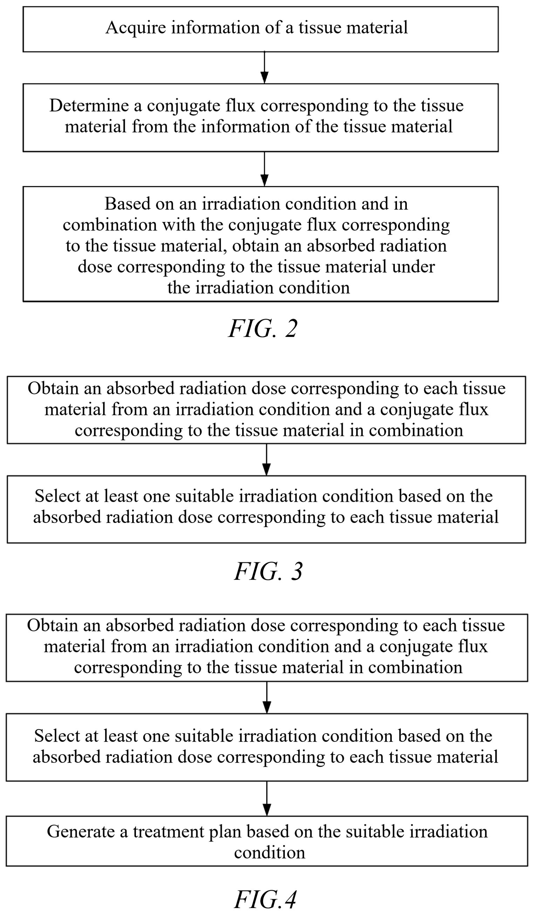

In view of this, in order to enable the quick evaluation of the absorbed radiation dose for the tissue material and the quick evaluation of the absorbed radiation doses for the tissue material under different irradiation conditions to facilitate the quick selection of a suitable irradiation condition, the embodiments of the present application provide a radiation therapy dose evaluation method. In an embodiment, as shown in FIG. 2, the method includes the following steps.

Information of a tissue material is acquired.

The information of the tissue material includes element information of the tissue material, such as an element composition and an element proportion of the tissue material. The human body tissue contains more than 60 kinds of elements, and more than 20 kinds thereof are essential elements, which are of great significance to maintain the normal physiological functions of a body. The elements with relatively high contents include carbon, hydrogen, oxygen, nitrogen, sodium, phosphorus, magnesium, potassium, calcium, etc. In addition, the blood tissue also contains a small amount of iron element, and the thyroid tissue contains a small amount of iodine element.

In addition, in some radiation therapy technologies, a targeted molecular drug is injected into the irradiated body before a therapeutic beam is controlled to irradiate the irradiated body, to utilize the selectivity of the targeted molecular drug to specifically accumulate a target element in certain tissues. For example, in boron neutron capture therapy, a boron-containing ($^{10}$B) drug selectively accumulates in tumor tissues. By utilizing the characteristic that boron ($^{10}$B) has a high capture cross-section for thermal neutrons, the purpose of locally killing tumor cells is achieved. In this application scenario, the element composition of the tissue material may also include the target element, and the element proportion of the tissue material may also include the proportion of the target element.

In radiation therapy technology, medical imaging such as Magnetic Resonance Imaging (MRI) or Computed Tomography (CT) can provide detailed tissue geometric structure information for the internal characteristics of the irradiated body. By way of example of Computed Tomography, tissue materials can be defined based on CT values, also referred to as Hounsfield Unit (HU) values. The tissue material with a fixed element composition ratio corresponds to a fixed range of HU values. For example, HU values between -100 and 20 may correspond to adipose tissue with a fixed element composition ratio, HU values between 20 and 100 may correspond to muscle tissue with a fixed element composition ratio, and HU values between - 951 and 120 may correspond to lung tissue with a fixed element composition ratio.

When the embodiments of the present application implement the conversion between the medical imaging data, the tissue material, and the information of the tissue material, there may be cases where some tissue materials cover a wide range of HU values, resulting in low dose calculation precision. In such cases, the tissue materials covering a wide range of HU values can be further divided. For example, lung tissue can be divided into low-density lung tissue and high-density lung tissue.

For radiation therapy technologies involving target elements, the embodiments of the present application can assume that the concentration distribution of the target element in some or all tissue materials is inhomogeneous, and acquire inhomogeneous target element distribution information through medical imaging such as Positron Emission Computed Tomography (PET). Thus, some or all tissue materials are further divided based on the target element distribution information. For example, a tumor tissue material is further divided into a plurality of tissue materials based on boron concentration to more accurately evaluate the absorbed radiation doses for tumor tissue materials with different boron concentrations.

For tumors at different locations, the embodiments of the present application require different tissue materials to be considered or subjected to dose calculation. For head and neck cancers, the tissue materials that usually need to be considered include skin, brain, brainstem, eye, mucosa, mandible, spinal cord, and tumor, etc. The embodiments of the present application can outline the geometric space of the tissue materials that need to be considered based on medical images of the irradiated body.

It should be noted that in the embodiments of the present application, acquiring the information of the tissue material specifically refers to acquiring information of a target tissue material for subsequent dose evaluation.

It should be noted that the embodiments of the present application do not specifically limit the definition of a tissue material, i.e., the conversion relationship between the medical imaging data and the tissue material. The tissue material requiring dose evaluation can be defined according to the actual accuracy requirements of dose evaluation and the tumor location.

It should be noted that the embodiments of the present application do not specifically limit the method for acquiring the information of the tissue material. For example, in proton radiation therapy, the HU values can be converted into corresponding tissue materials via TOPAS software based on a specific relationship, and then tissue material element information predefined by default in the TOPAS software can be acquired. For example, in boron neutron capture therapy, the information of the tissue material can be acquired from a material template library of MCNP software and PET medical imaging data or blood boron concentration in combination.

A conjugate flux corresponding to the tissue material is determined from the information of the tissue material.

The conjugate flux corresponding to the tissue material includes distribution information of the conjugate flux within a geometric space of the tissue material.

As an example, after acquiring the information of the tissue material, the conjugate flux corresponding to the tissue material is determined from the element information in the information of the tissue material, where the conjugate flux is irrelevant to the variable source term S, i.e., the parameters of the radiation. Therefore, when calculating the dose distributions under a plurality of irradiation conditions using the radiation therapy dose evaluation method according to the embodiments of the present application, the conjugate flux corresponding to one type of tissue material only needs to be calculated once. That is, the number of calculations of the conjugate flux is related to only the number of types of tissue materials that need to be considered, and is irrelevant to the number of irradiation conditions.

Based on an irradiation condition and in combination with the conjugate flux corresponding to the tissue material, an absorbed radiation dose corresponding to the tissue material under the irradiation condition is obtained.

The irradiation condition includes the irradiation direction in which radioactive particles form a beam, irradiation position, and beam parameters, etc. Essentially, different irradiation conditions correspond to different source terms $S$. Under different irradiation conditions, different radioactive particles exhibit different distribution states, and the source term $S$ can be represented by a distribution function of the particles.

As an example, after determining the conjugate flux corresponding to the tissue material, based on the irradiation condition and in combination with the conjugate flux corresponding to the tissue material, the absorbed radiation dose corresponding to the tissue material under the irradiation condition can be determined. For example, based on the conjugate flux corresponding to the tumor tissue and a given irradiation condition, the absorbed radiation dose corresponding to the tumor tissue under the irradiation condition is obtained.

In radiation therapy technology, it is often necessary to calculate the dose distributions of the tissue material under numerous different irradiation conditions. For example, to select a suitable irradiation condition from 100 irradiation conditions, it is required to calculate the doses $D$ of radioactive particles corresponding to these 100 irradiation conditions. When the flux $\varnothing$ is used to calculate the dose $D$, the flux $\varnothing$ is calculated using Formula 2. As can be seen from Formula 2, the flux $\varnothing$ is related to the source term S, which means that the flux $\varnothing$ needs to be calculated for each irradiation condition. Thus, the flux $\varnothing$ must be calculated 100 times to obtain the doses $D$ corresponding to these 100 irradiation conditions. In contrast, when the conjugate flux provided in the embodiments of the present application is used to calculate the dose $D$, since the conjugate flux is irrelevant to the source term S, the number of calculations for the conjugate flux is related to only the count target of types of tissue materials that need to be considered. Therefore, the conjugate flux corresponding to the tissue material is the same for different irradiation conditions. Thus, when the conjugate flux provided in the embodiments of the present application is used to calculate the dose $D$, it is only necessary to calculate the conjugate flux several times, and then use the 100 irradiation conditions to calculate the dose $D$ corresponding to the tissue material under each irradiation condition. The specific number of calculations depends on the number of types of tissue materials that need to be considered, which is generally the number of tumor tissues and normal tissues. Obviously, the number of tissue materials is much smaller than the number of irradiation conditions that need to be considered. Moreover, the calculation of the flux $\varnothing$ is very complex, and the calculation of the conjugate flux is simpler than that of the flux $\varnothing$. In addition, the calculation of the dose $D$ from the irradiation condition and the conjugate flux in combination is also relatively simple. Therefore, compared with using the flux $\varnothing$ to calculate the doses $D$ under a plurality of irradiation conditions, using the conjugate flux to calculate the doses $D$ under a plurality of irradiation conditions is significantly faster in speed.

In the radiation therapy dose evaluation method described above, the information of the tissue material is first acquired, the conjugate flux corresponding to the tissue material is determined from the information of the tissue material, and based on the irradiation condition and in combination with the conjugate flux corresponding to the tissue material, the absorbed radiation dose corresponding to the tissue material under the irradiation condition is obtained. Since the calculation of the conjugate flux is relatively simple, and the calculation of the absorbed radiation dose from the irradiation condition and the conjugate flux in combination is also relatively simple, the use of the method described above enables the quick evaluation of the absorbed radiation dose corresponding to the tissue material. Moreover, when the method described above is used to evaluate the dose distributions under a plurality of irradiation conditions, since the conjugate flux is irrelevant to the source term S, the number of calculations for the conjugate flux is related to only the count target of tissue materials that need to be considered. Therefore, regardless of changes in irradiation conditions, the conjugate flux corresponding to a tissue material is fixed. Thus, by using the radiation therapy dose evaluation method described above, it is only necessary to calculate the conjugate flux several times to evaluate the dose distributions under a plurality of irradiation conditions, which improves the efficiency of dose evaluation under a plurality of irradiation conditions.

In one embodiment, determining the conjugate flux corresponding to the tissue material from the information of the tissue material includes the following steps.

A flux-dose conversion factor corresponding to the tissue material is determined from element information of the tissue material in the information of the tissue material.

The flux-dose conversion factor (*Kerma*) of a certain type of tissue material is determined by the element composition and element proportion of the tissue material. Based on the particle energy, the flux-dose conversion factor of each nuclide at the corresponding energy can be found in a cross-section database.

In an implementation, first, a density of each nuclide in the tissue material is acquired from the element composition and the element proportion in the tissue material, and a flux-dose conversion factor corresponding to each nuclide is acquired. The flux-dose conversion factor corresponding to the tissue material is then determined from the flux-dose conversion factor corresponding to each nuclide and the density of each nuclide in combination.

In an implementation, the flux-dose conversion factor corresponding to the tissue material is determined from the flux-dose conversion factor corresponding to each nuclide and the density of each nuclide by

$$Kerma = \sum \rho_i \cdot Kerma_i; \text{ (Formula 3)}$$

where $\rho_i$ represents a density of an *i*-th nuclide, and *Kerma_i* represents a flux-dose conversion factor corresponding to the *i*-th nuclide.

The conjugate flux corresponding to the tissue material is determined from the flux-dose conversion factor corresponding to the tissue material.

In an implementation, based on the flux-dose conversion factor *Kerma* corresponding to the tissue material and in combination with the aforementioned Formula 2, a conjugate equation is built with the flux-dose conversion factor *Kerma* corresponding to the tissue material as the source term. The conjugate flux corresponding to the tissue material is determined using this conjugate equation, which is as follows:

$$\emptyset^*(P_1) = Kerma(P_1) + \int K^*(P_2 \to P_1)\emptyset^*(P_2)dP_2; \text{ (Formula 4)}$$

where $\emptyset^*$ represents a conjugate flux, $P_1$ and $P_2$ represent different particle state parameters, $\emptyset^*(P_1)$ represents a conjugate flux of particles in the state $P_1$, *Kerma* represents a flux-dose conversion factor, and $K^*$ represents a conjugate kernel parameter in the nuclear reaction database.

The transport kernel $K$ and the conjugate kernel $K^*$ satisfy the following relationship:

$$K(P_2 \to P_1) = K^*(P_1 \to P_2); \text{ (Formula 5)}$$

As can be seen, $K^*(P_2 \to P_1)$ in Formula 4 above is equal to $K(P_1 \to P_2)$, i.e., the probability that a particle transitions from the state $P_1$ to state $P_2$ by a collision reaction.

In this embodiment, the flux-dose conversion factor corresponding to the tissue material is determined from the element information of the tissue material in the information of the tissue material, and the conjugate flux corresponding to the tissue material is determined from the flux-dose conversion factor corresponding to the tissue material. Referring to Formula 4, the conjugate flux is irrelevant to the source term $S$, and is related to the flux-dose conversion factor *Kerma*, and the flux-dose conversion factor *Kerma* corresponds to the tissue material. Nuclides in different tissue materials have different densities, and different nuclides correspond to different *Kerma* values. A *Kerma* value corresponding to the tissue material can be obtained from the density of each nuclide in the tissue material and the *Kerma* value corresponding to each nuclide in combination. Then, according to Formula 4, the conjugate flux corresponding to the tissue material is determined from the *Kerma* value corresponding to the tissue material.

In an embodiment, based on the irradiation condition and in combination with the conjugate flux corresponding to the tissue material, obtaining the absorbed radiation dose corresponding to the tissue material under the irradiation condition includes:

determining a source term distribution corresponding to the irradiation condition based on the irradiation condition, where the source term distribution is the source term $S$ and is used to characterize a distribution function of a particle source under the irradiation condition; and
acquiring the absorbed radiation dose corresponding to the tissue material from of the source term distribution and the conjugate flux corresponding to the tissue material.

In combination with Formula 2 and Formula 4, the inner product of two sides of Formula 2 with Ø* is computed, and the inner product of two sides of Formula 4 with Ø is computed, resulting in:

$$\int \emptyset(P_1)\emptyset^*(P_1)\,dP_1 = \int S(P_1)\,\emptyset^*(P_1)dP_1 + \int\int K(P_2 \to P_1)\emptyset(P_2)\emptyset^*(P_1)dP_2dP_1 ; \text{(Formula 6)}$$

and

$$\int \emptyset(P_1)\emptyset^*(P_1)\,dP_1 = \int Kerma(P_1)\,\emptyset(P_1)dP_1 + \int\int K^*(P_2 \to P_1)\emptyset(P_1)\emptyset^*(P_2)dP_2\,dP_1 \quad ;$$
(Formula 7)

According to Formula 5, the two double integrals on the right-hand side of the equal sign in Formula 6 and Formula 7 are equal. Elimination of these equal parts yields:

$$\int S(P_1)\emptyset^*(P_1)dP_1 = \int Kerma(P_1)\,\emptyset(P_1)dP_1 ; \text{(Formula 8)}$$

Combining Formula 1, Formula 2, Formula 4, and Formula 8 gives:

$$D = <\emptyset, \; kerma> = <\emptyset^*, \; S>; \text{(Formula 9)}$$

Formula 9 indicates that the dose can be obtained by means of the inner product of the flux Ø and the flux-dose conversion factor *Kerma,* or by means of the inner product of the conjugate flux Ø* and the source term *S.*

In this embodiment, the source term distribution function corresponding to the irradiation condition is determined from the irradiation condition, and then the inner product of the source term distribution function and the conjugate flux corresponding to the tissue material is computed to obtain the absorbed radiation dose corresponding to a region of interest. The calculation of the flux Ø is complex and time-consuming, the calculation of the conjugate flux Ø* is simpler than that of the flux Ø, and the calculation of an inner product is also relatively simple. Therefore, the use of the method described above enables the quick evaluation of the absorbed radiation dose corresponding to the tissue material. Moreover, when evaluating the dose distributions under a plurality of irradiation conditions (for example, to select a suitable irradiation condition from 100 irradiation conditions), if the conjugate flux Ø* provided in the embodiments of the present application is used to calculate the dose *D,* it is only necessary to calculate the conjugate flux Ø* several times (the specific number of times depends on the count target of tissue materials that need to be considered), and then compute the inner product of the conjugate flux Ø* and the source term distribution function 100 times to obtain the doses *D* corresponding to different source terms. This can significantly save the time for dose evaluation and improve the efficiency of dose evaluation.

Based on the same inventive concept, the embodiments of the present application further provide an irradiation condition selection method. The implementation solutions for solving problems provided by this irradiation condition selection method are similar to those described in the radiation therapy dose evaluation method described above. Therefore, the definitions above on the radiation therapy dose evaluation method can be referred to for the specific definitions in the following one or more embodiments of the irradiation condition selection method, which will not be repeated here.

In an embodiment, as shown in FIG. 3, an irradiation condition selection method is provided. The method includes the following steps.

An absorbed radiation dose corresponding to each tissue material is obtained from an irradiation condition and a conjugate flux corresponding to the tissue material in combination.

When selecting a suitable irradiation condition, it is required that high-dose radiation is delivered to a tumor target area as much as possible while minimizing damage to surrounding organs (healthy tissues). To better control the dose distribution within each organ or target area, the suitable irradiation condition is selected such that the radiation kills tumor cells while minimizing damage to surrounding normal tissues. In the embodiments of the present application, the tissue materials may include tumor tissue and at least one important organ tissue located in the surrounding area.

In an implementation, a plurality of irradiation conditions can be predetermined with the experience of a physicist based on tumor type, location and size. Sampling from the plurality of irradiation conditions, that is, selecting a certain irradiation condition, can be performed randomly or in a preset order. The absorbed radiation dose corresponding to each tissue material under the irradiation condition is obtained from the irradiation condition and the conjugate flux corresponding to the tissue material in combination.

At least one suitable irradiation condition is selected based on the absorbed radiation dose corresponding to each tissue material.

In an implementation, if the absorbed radiation dose corresponding to each tissue material meets a preset condition, the irradiation condition is accepted; and if the absorbed radiation dose corresponding to each tissue material does not meet the preset condition, an irradiation condition is sampled again, and the absorbed radiation dose corresponding to each tissue material under the re-sampled irradiation condition is evaluated.

As an example, the tissue material includes a tumor tissue material and an important organ tissue material, and if the absorbed radiation dose corresponding to the tumor tissue material is greater than a first preset threshold, and the absorbed radiation dose corresponding to the important organ tissue material is less than a second preset threshold, the irradiation condition is accepted as a suitable irradiation condition.

In an implementation, a plurality of irradiation conditions are screened for optimal ones based on the absorbed radiation dose corresponding to each tissue material, so as to select at least one suitable irradiation condition.

As an example, for the absorbed radiation dose corresponding to each tissue material under each irradiation condition, the irradiation conditions meeting the preset condition are first screened out, and then the plurality of irradiation conditions meeting the preset condition are sorted and screened for optimal ones. The optimal ones can be determined by comparing the absorbed radiation dose corresponding to the tumor tissue under each irradiation condition and comparing the absorbed radiation doses corresponding to other important organ tissue materials under each irradiation condition. Alternatively, different weights can be assigned to different tissue materials according to actual situations for screening, which is not specifically limited in the embodiments of the present application.

It should be noted that the screening may involve selecting one optimal irradiation condition or a plurality of sub-optimal irradiation conditions, and the one optimal irradiation condition or the plurality of sub-optimal irradiation conditions are used as suitable irradiation conditions. Alternatively, when a plurality of sub-optimal irradiation conditions are selected, a Monte Carlo simulation program can be used to calculate more accurate dose distributions under the plurality of sub-optimal irradiation conditions based on the fluxes corresponding to the plurality of sub-optimal irradiation conditions, and then a comparison is performed to determine the suitable irradiation condition.

In this embodiment, the absorbed radiation dose corresponding to each tissue material is obtained from the irradiation condition and the conjugate flux corresponding to the tissue material in combination, and at least one suitable irradiation condition is selected based on the absorbed radiation dose corresponding to each tissue material. Since the conjugate flux is irrelevant to the source term S, the number of calculations for the conjugate flux is related to only the count target of tissue materials that need to be considered. Therefore, regardless of changes in irradiation conditions, the conjugate flux corresponding to a tissue material remains fixed. Thus, by using the irradiation condition selection method described above, it is only necessary to calculate the conjugate flux several times to evaluate the absorbed radiation doses for each tissue material under a plurality of irradiation conditions, which improves the efficiency of dose calculation under a plurality of irradiation conditions, and enables the quick evaluation of the absorbed radiation doses corresponding to each tissue material under different irradiation conditions, so as to facilitate the quick selection of a suitable irradiation condition from the plurality of irradiation conditions.

In an embodiment, the method includes, prior to obtaining the absorbed radiation dose corresponding to each tissue material from the irradiation condition and the conjugate flux corresponding to the tissue material in combination, based on at least one preset region of interest, determining a tissue material corresponding to the region of interest.

The region of interest (ROI) may be a target region for particle deposition, such as a tumor region requiring treatment, or may be a protection region around the target region, such as a region where an important organ tissue is located. The important organ tissue may be a critical organ such as eye and liver, or an important tissue, such as bone tissue and brain tissue.

In the embodiments of the present application, at least one ROI region to be defined for different tumor types can be preset. For example, the ROI types requiring attention during brain tumor treatment may include air, skin, carotid, mucosa, brain, eyeball, eyelens, gland, skeleton, soft tissue, or brain tumor.

Based on the medical imaging data (e.g., HU values), the corresponding ROI boundaries can be automatically defined, automatically read, or manually defined by an operator. Through the definition of ROI boundaries, a three-dimensional medical imaging geometric model of the irradiated body is divided into corresponding ROI regions, that is, voxel grids are assigned to the corresponding ROI regions.

In the embodiments of the present application, after presetting at least one ROI region, for each ROI region, the tissue material corresponding to the ROI region is determined. For example, the HU value range corresponding to each ROI region, one or more tissue material types corresponding to different ROI regions, the HU values corresponding to each tissue material, and the element information corresponding to each tissue material are preset in the material template library.

As an example, some ROI region covers a narrow HU value range, and one ROI region may correspond to one tissue material.

As an example, some ROI region covers a wide HU value range, and such a ROI region can be further divided into different tissue material types based on different HU value ranges, resulting in a plurality of tissue materials corresponding to the ROI region.

It should be noted that in the radiation therapy application scenario involving target elements, the concentration distribution of the target element in the ROI region is assumed to be homogeneous in this embodiment.

In this embodiment, by selecting at least one preset region of interest and determining the type of tissue material corresponding to the region of interest based on the tissue density in the medical imaging data, one or more tissue materials corresponding to the region of interest are obtained, enabling more precise evaluation of the absorbed radiation dose for the tissue material.

In another embodiment, the method includes, prior to obtaining the absorbed radiation dose corresponding to each tissue material from the irradiation condition and the conjugate flux corresponding to the tissue material in combination, based on at least one preset region of interest and in combination with a concentration distribution of a target element in the region of interest, determining a tissue material corresponding to the region of interest.

This embodiment is applied to a radiation therapy scenario involving target elements, where the concentration distribution of the target element in the ROI region is assumed to be partially inhomogeneous or fully inhomogeneous.

As an example, after presetting the ROI region, the initial type of tissue material corresponding to the ROI region can be first determined from the HU value of the ROI region, and then part or all of the tissue material in the initial type of tissue material can be further divided based on the inhomogeneous concentration distribution information of the target element. For example, in boron neutron capture therapy, a ROI region is determined to correspond to one type of tissue material based on the HU value, and the tissue material is tumor tissue. Further, the inhomogeneous boron nuclide concentration distribution information in the ROI region can be acquired through medical imaging such as PET, and based on the inhomogeneous boron nuclide concentration distribution information, the ROI region can be further divided into a first tumor tissue material, a second tumor tissue material, a third tumor tissue material, etc.

In this embodiment, the tissue material corresponding to the region of interest is determined from the concentration distribution of the target element in the region of interest in combination. Since in the calculation of the absorbed radiation dose for the tissue material, the target element also has a corresponding flux-dose conversion factor *Kerma* value, the inhomogeneous distribution of the target element may affect the flux-dose conversion factor *Kerma* value corresponding to the tissue material, thereby causing deviations in the absorbed radiation dose for the tissue material. Therefore, in this embodiment, the tissue material in the region of interest can be further divided based on the concentration distribution of the target element, so as to evaluate the absorbed radiation dose for the tissue material more precisely.

In an embodiment, selecting the suitable irradiation condition based on the absorbed radiation dose corresponding to each tissue material includes:

determining an absorbed radiation dose for the region of interest from absorbed radiation doses corresponding to all tissue materials in the region of interest.

In case that a region of interest is selected prior to obtaining the absorbed radiation dose corresponding to each tissue material, a suitable irradiation condition can be selected based on a total absorbed radiation dose within the region of interest.

As an example, when there is only one type of tissue material in the region of interest, the total absorbed radiation dose in the region of interest is the absorbed dose for the tissue material corresponding to the region of interest.

As an example, when the region of interest includes multiple types of tissue materials, the absorbed radiation dose for the region of interest can be obtained by dividing a total deposited energy for all tissue materials by the mass of the region of interest, that is, a total average dose for the region of interest is used as the absorbed radiation dose for the region of interest. The absorbed radiation dose for the region of interest can be calculated by:

$$D = \frac{\sum_{i=1}^{N} E_{i\_deposite}}{\sum_{i=1}^{N} M_i} = \frac{\sum_{i=1}^{N} (D_i \cdot M_i)}{\sum_{i=1}^{N} M_i}; \text{ (Formula 10)}$$

where $E_{i\_deposite}$ represents the total deposited energy corresponding to the *i*-th type of tissue material, $M_i$ represents the mass of the *i*-th type of tissue material, and $D_i$ represents the absorbed radiation dose corresponding to the *i*-th type of tissue material.

A suitable irradiation condition is selected based on the absorbed radiation dose for the region of interest.

In this embodiment, after selecting the region of interest and dividing the tissue materials within the region of interest, the absorbed radiation dose corresponding to each tissue material is determined from the conjugate flux and irradiation condition; the absorbed radiation dose for the region of interest is determined from the absorbed radiation doses corresponding to all the tissue materials in the region of interest and the mass of all the tissue materials; and the suitable irradiation condition is selected by determining whether the absorbed radiation dose for the region of interest meets the preset condition.

Based on the same inventive concept, the embodiments of the present application further provide a treatment plan generation method. This treatment plan generation method uses the irradiation condition selection method described above to generate a treatment plan. Specifically, an absorbed radiation dose corresponding to each tissue material is obtained from an irradiation condition and a conjugate flux corresponding to the tissue material in combination; at least one suitable irradiation condition is selected based on the absorbed radiation dose corresponding to each tissue material; and a treatment plan is generated based on the suitable irradiation condition.

The steps of obtaining the absorbed radiation dose corresponding to each tissue material from the irradiation condition and the conjugate flux corresponding to the tissue material in combination, and selecting at least one suitable irradiation condition based on the absorbed radiation doses corresponding to each tissue material are the same as the implementation solutions recorded in the irradiation condition selection method described above. Therefore, the definitions above on the irradiation condition selection method can be referred to for the specific definitions in the following one or more embodiments of the treatment plan generation method, which will not be repeated here.

In an embodiment, as shown in FIG. 4, the treatment plan generation method includes: obtaining an absorbed radiation dose corresponding to each tissue material from an irradiation condition and a conjugate flux corresponding to the tissue material in combination; and selecting at least one suitable irradiation condition based on the absorbed radiation dose corresponding to each tissue material; and generating a treatment plan based on the suitable irradiation condition.

In an embodiment, selecting at least one suitable irradiation condition based on the absorbed radiation dose corresponding to each tissue material includes: accepting the irradiation condition if the absorbed radiation dose corresponding to each tissue material meets a preset condition.

In an embodiment, the tissue material includes at least a tumor tissue material and an important organ tissue material, and accepting the irradiation condition if the absorbed radiation dose corresponding to each tissue material meets the preset condition includes: if the absorbed radiation dose corresponding to the tumor tissue material is greater than a first preset threshold, and the absorbed radiation dose corresponding to the important organ tissue material is less than a second preset threshold, accepting the irradiation condition.

In an embodiment, the method includes, prior to obtaining the absorbed radiation dose corresponding to each tissue material from the irradiation condition and the conjugate flux corresponding to the tissue material in combination, based on at least one preset region of interest, determining a tissue material corresponding to the region of interest.

In an embodiment, the method includes, prior to obtaining the absorbed radiation dose corresponding to each tissue material from the irradiation condition and the conjugate flux corresponding to the tissue material in combination, based on at least one preset region of interest and in combination with a concentration distribution of a target element in the region of interest, determining a tissue material corresponding to the region of interest.

In an embodiment, selecting the suitable irradiation condition based on the absorbed radiation dose corresponding to each tissue material includes: determining an absorbed radiation dose for the region of interest from absorbed radiation doses corresponding to all tissue materials in the region of interest; and selecting the suitable irradiation condition based on the absorbed radiation dose for the region of interest.

In an embodiment, generating the treatment plan based on the suitable irradiation condition includes: determining a corresponding flux based on the suitable irradiation condition, and performing dose distribution calculation using a Monte Carlo simulation program based on the flux.

It should be understood that although the steps in the flowcharts involved in the above embodiments are shown in sequence as indicated by the arrows, these steps are not necessarily performed in sequence in the order indicated by the arrows. Unless explicitly described herein, the performance of these steps is not limited to a strict order, instead, the steps may be performed in another order. Furthermore, at least some of the steps in the flowcharts involved in the above embodiments may include a plurality of steps or stages. These steps or stages are not necessarily completed at the same time, but may be performed at different moments. These steps or stages are not necessarily performed in sequence, but may be performed in turn or alternately with other steps or at least some of steps or stages in other steps.

Based on the same inventive concept, the embodiments of the present application further provide a treatment plan generation apparatus for implementing the treatment plan generation method described above. The implementation solutions for solving problems provided by this treatment plan generation apparatus are similar to those described in the treatment plan generation method described above. Therefore, the definitions above on the treatment plan generation method can be referred to for the specific definitions in the following one or more embodiments of the treatment plan generation apparatus, which will not be repeated here.

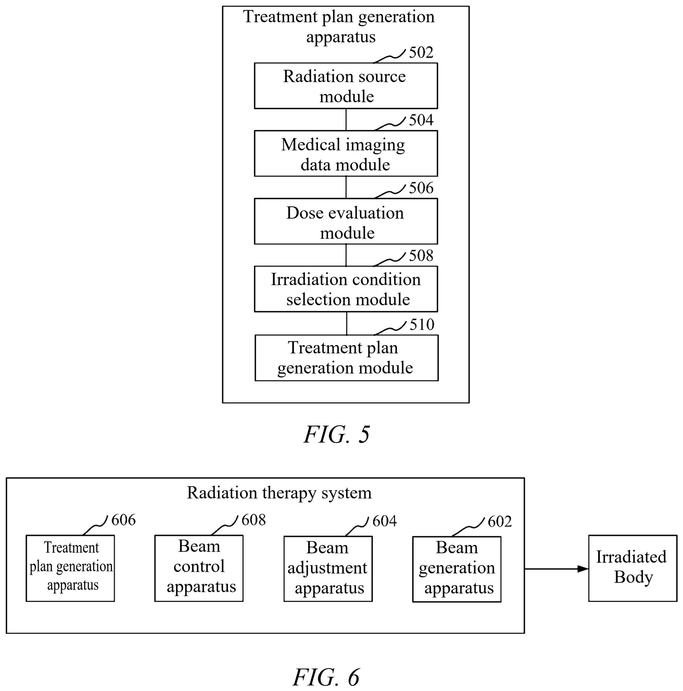

In an embodiment, as shown in FIG. 5, a treatment plan generation apparatus is provided. The treatment plan generation apparatus includes a radiation source module 502, a medical imaging data module 504, a dose evaluation module 506, an irradiation condition selection module 508, and a treatment plan generation module 510.

The radiation source module 502 is configured to establish source term information based on different irradiation conditions.

The medical imaging data module 504 is configured to acquire information of a tissue material based on medical imaging data and establish a three-dimensional voxel tissue model.

The dose evaluation module 506 is configured to determine a conjugate flux corresponding to the tissue material from the information of the tissue material and obtain an absorbed radiation dose corresponding to each tissue material from the source term information and the conjugate flux corresponding to the tissue material in combination.

The irradiation condition selection module 508 is configured to select at least one suitable irradiation condition based on the absorbed radiation dose corresponding to each tissue material.

The treatment plan generation module 510 is configured to generate a treatment plan based on the suitable irradiation condition.

All modules in the treatment plan generation apparatus described above can be fully or partially implemented through software, hardware, or a combination thereof. Each of the above modules can be embedded in or independent of a processor in a computer device in the form of hardware, or stored in a memory of the computer device in the form of software, so that the processor can invoke them to execute the operations corresponding to each of the above modules.

Based on the same inventive concept, the embodiments of the present application further provide a radiation therapy system. The implementation solutions for solving problems provided by this radiation therapy system are similar to those described in the treatment plan generation method or treatment plan generation apparatus described above. Therefore, the definitions above on the treatment plan generation method or treatment plan generation apparatus can be referred to for the specific definitions in the following one or more embodiments of the radiation therapy system, which will not be repeated here.

In an embodiment, as shown in FIG. 6, a radiation therapy system is provided. The radiation therapy system includes a beam generation apparatus 602, a beam adjustment apparatus 604, a treatment plan generation apparatus 606, and a beam control apparatus 608.

The beam generation apparatus 602 is configured to generate a therapeutic beam.

The beam adjustment apparatus 604 is configured to adjust the therapeutic beam generated by the beam generation apparatus 602.

The treatment plan generation apparatus 606 is configured to establish source term information based on different irradiation conditions, acquire information of a tissue material based on medical imaging data, establish a three-dimensional voxel tissue model, determine a conjugate flux corresponding to the tissue material from the information of the tissue material, obtain an absorbed radiation dose corresponding to each tissue material from the source term information and the conjugate flux corresponding to the tissue material in combination, select at least one suitable irradiation condition based on the absorbed radiation doses corresponding to each tissue material, and generate a treatment plan based on the suitable irradiation condition.

The beam control apparatus 608 is configured to retrieve the corresponding treatment plan from the treatment plan generation apparatus 606 and control the beam generation apparatus 602 to perform the treatment plan.

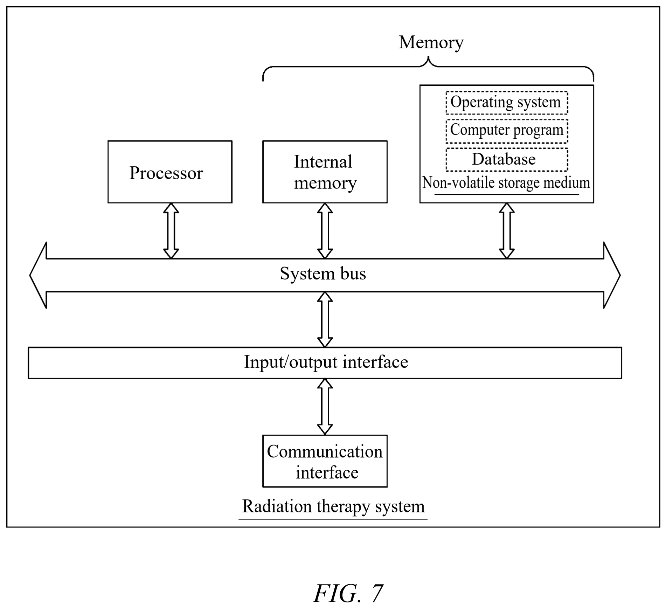

Based on the same inventive concept, the embodiments of the present application further provide another radiation therapy system, which may be a server, and an internal structure of which may be shown in FIG. 7. The computer device includes a processor, a memory, an input/output (I/O) interface, and a communication interface. The processor, the memory, and the input/output interface are connected via a system bus, and the communication interface is connected to the system bus via the input/output interface. The processor of the computer device is configured to provide computing and control capabilities. The memory of the computer device includes a non-volatile storage medium and an internal memory. The non-volatile storage medium stores an operating system, a computer program, and a database. The internal memory provides an environment for the operation of the operating system and the computer program in the non-volatile storage medium. The database of the computer device is configured to store data such as information of a tissue material. The input/output interface of the computer device is configured to exchange information between the processor and external devices. The communication interface of the computer device is configured for communicating with external terminals via network connections. The computer program, when executed by the processor, causes the radiation therapy dose evaluation method, the irradiation condition selection method, or the treatment plan generation method to be implemented.

Those skilled in the art will understand that the structure shown in FIG. 7 is merely a block diagram of part of the structure related to the solution of the present application, and does not constitute a limitation on the radiation therapy system to which the solution of the present application is applied. A specific radiation therapy system may include more or fewer components than those shown in the figure, or combine some components, or have a different arrangement of components.

In an embodiment, a radiation therapy system is provided. The radiation therapy system includes a memory and a processor, where a computer program is stored in the memory, and the processor, when executing the computer program, causes the steps in each of the above method embodiments to be implemented.

In an embodiment, a computer-readable storage medium is provided, on which a computer program is stored, where the computer program, when executed by a processor, causes the steps in each of the above method embodiments to be implemented.

Those of ordinary skill in the art will understand that all or part of the processes in the methods of the above embodiments can be completed by instructing relevant hardware through a computer program. The computer program can be stored in a non-volatile computer-readable storage medium. The computer program, when executed, can include the processes of the above method embodiments. Any reference to the memory, the database or other media used in the embodiments provided in the present application may include at least one of non-volatile and volatile memories. The non-volatile memory may include a read-only memory (ROM), a magnetic tape, a floppy disk, a flash memory, an optical memory, a high-density embedded non-volatile memory, a resistive random access memory (ReRAM), a magnetoresistive random access memory (MRAM), a ferroelectric random access memory (FRAM), a phase change memory (PCM), a graphene memory, and the like. The volatile memory may include a random access memory (RAM), an external cache memory, and the like. By way of illustration rather than limitation, the RAM may be in various forms, such as a static random access memory (SRAM) or a dynamic random access memory (DRAM).

The technical features of the above embodiments may be combined arbitrarily. For the purpose of brevity of description, all the possible combinations of the technical features in the above embodiments are not described. However, as long as there is no contradiction between the combinations of these technical features, they shall all fall within the scope of the specification.

The embodiments described above merely illustrate several implementations of the present application and are described relatively specifically and in detail, but should not be construed as limiting the patent scope of the present application. It should be noted that several variations and improvements may also be made by those of ordinary skill in the art without departing from the concept of the present application, and those modifications and improvements shall all fall within the scope of protection of the present application. Therefore, the scope of protection of the present application shall be subject to the appended claims.

**Claims**

1. A radiation therapy dose evaluation method, **characterized by** comprising:

   acquiring information of a tissue material;
   determining a conjugate flux corresponding to the tissue material from the information of the tissue material; and
   based on an irradiation condition and in combination with the conjugate flux corresponding to the tissue material, obtaining an absorbed radiation dose corresponding to the tissue material under the irradiation condition.

2. The method according to claim 1, **characterized in that** determining the conjugate flux corresponding to the tissue material from the information of the tissue material comprises:

   determining a flux-dose conversion factor corresponding to the tissue material from element information of the tissue material in the information of the tissue material; and
   determining the conjugate flux corresponding to the tissue material from the flux-dose conversion factor corresponding to the tissue material.

3. The method according to claim 2, **characterized in that** the element information comprises an element composition and an element proportion, and determining the flux-dose conversion factor corresponding to the tissue material from the element information of the tissue material in the information of the tissue material comprises:

   acquiring a density of each nuclide in the tissue material from the element composition and the element proportion;
   acquiring a flux-dose conversion factor corresponding to each nuclide; and
   determining the flux-dose conversion factor corresponding to the tissue material from the flux-dose conversion factor corresponding to each nuclide and the density of each nuclide in combination.

4. The method according to claim 3, **characterized in that** the flux-dose conversion factor corresponding to the tissue material is determined from the flux-dose conversion factor corresponding to each nuclide and the density of each nuclide in combination by

$$Kerma = \sum \rho_i \cdot Kerma_i;$$

wherein $\rho_i$ represents a density of an i-th nuclide, and $Kerma_i$ represents a flux-dose conversion factor corresponding to the i-th nuclide.

5. The method according to claim 2, **characterized in that** the conjugate flux corresponding to the tissue material is determined from the flux-dose conversion factor corresponding to the tissue material by

$$\emptyset^*(P_1) = Kerma(P_1) + \int K^*(P_2 \rightarrow P_1)\emptyset^*(P_2)dP_2;$$

wherein $\emptyset^*$ represents a conjugate flux, $P_1$ and $P_2$ represent different particle state parameters, $\emptyset^*(P_1)$ represents a conjugate flux of particles in the state $P_1$, *Kerma* represents a flux-dose conversion factor, $K^*$ represents a conjugate kernel, and $K^*(P_2 \rightarrow P_1)$ represents a probability that a particle transitions from the state $P_1$ to the state $P_2$ by a collision reaction.

6. The method according to claim 1, **characterized in that** based on the irradiation condition and in combination with the conjugate flux corresponding to the tissue material, obtaining the absorbed radiation dose corresponding to the tissue material under the irradiation condition comprises:

   determining a source term distribution corresponding to the irradiation condition based on the irradiation condition, wherein the source term distribution is used to characterize a distribution function of a particle source under the irradiation condition; and
   acquiring the absorbed radiation dose corresponding to the tissue material from the source term distribution and the conjugate flux corresponding to the tissue material.

7. An irradiation condition selection method, **characterized by** comprising:

   obtaining an absorbed radiation dose corresponding to each tissue material from an irradiation condition and a conjugate flux corresponding to the tissue material in combination; and
   selecting at least one suitable irradiation condition based on the absorbed radiation dose corresponding to each tissue material.

8. The method according to claim 7, **characterized in that** selecting at least one suitable irradiation condition based on the absorbed radiation dose corresponding to each tissue material comprises:
   accepting the irradiation condition if the absorbed radiation dose corresponding to each tissue material meets a preset condition.

9. The method according to claim 8, **characterized in that** the tissue material comprises at least a tumor tissue material and an important organ tissue material, and accepting the irradiation condition if the absorbed radiation dose corresponding to each tissue material meets the preset condition comprises:
   if the absorbed radiation dose corresponding to the tumor tissue material is greater than a first preset threshold, and the absorbed radiation dose corresponding to the important organ tissue material is less than a second preset threshold, accepting the irradiation condition.

10. The method according to claim 7, **characterized in that** the method comprises, prior to obtaining the absorbed radiation dose corresponding to each tissue material from the irradiation condition and the conjugate flux corresponding to the tissue material in combination,
    based on at least one preset region of interest, determining a tissue material corresponding to the region of interest.

11. The method according to claim 7, **characterized in that** the method comprises, prior to obtaining the absorbed radiation dose corresponding to each tissue material from the irradiation condition and the conjugate flux corresponding to the tissue material in combination,
    based on at least one preset region of interest and in combination with a concentration distribution of a target element in the region of interest, determining a tissue material corresponding to the region of interest.

12. The method according to claim 10 or 11, **characterized in that** selecting the suitable irradiation condition based on the absorbed radiation dose corresponding to each tissue material comprises:

    determining an absorbed radiation dose for the region of interest from absorbed radiation doses corresponding to all tissue materials in the region of interest; and
    selecting the suitable irradiation condition based on the absorbed radiation dose for the region of interest.

13. A treatment plan generation apparatus, **characterized by** comprising:

a radiation source module configured to establish source term information based on different irradiation conditions;
a medical imaging data module configured to acquire information of a tissue material based on medical imaging data and establish a three-dimensional voxel tissue model;
a dose evaluation module configured to determine a conjugate flux corresponding to the tissue material from the information of the tissue material, and obtain an absorbed radiation dose corresponding to each tissue material from the source term information and the conjugate flux corresponding to the tissue material in combination;
an irradiation condition selection module configured to select at least one suitable irradiation condition based on the absorbed radiation dose corresponding to each tissue material; and
a treatment plan generation module configured to generate a treatment plan based on the suitable irradiation condition.

14. A radiation therapy system, **characterized by** comprising:

a beam generation apparatus configured to generate a therapeutic beam;
a beam adjustment apparatus configured to adjust the therapeutic beam generated by the beam generation apparatus;
a treatment plan generation apparatus configured to establish source term information based on different irradiation conditions, acquire information of a tissue material based on medical imaging data, establish a three-dimensional voxel tissue model, determine a conjugate flux corresponding to the tissue material from the information of the tissue material, obtain an absorbed radiation dose corresponding to each tissue material from the source term information and the conjugate flux corresponding to the tissue material in combination, select at least one suitable irradiation condition based on the absorbed radiation dose corresponding to each tissue material, and generate a treatment plan based on the suitable irradiation condition; and
a beam control apparatus configured to retrieve a corresponding treatment plan from the treatment plan generation apparatus and control the beam generation apparatus to perform the treatment plan.

*FIG. 1*

Acquire information of a tissue material

Determine a conjugate flux corresponding to the tissue material from the information of the tissue material

Based on an irradiation condition and in combination with the conjugate flux corresponding to the tissue material, obtain an absorbed radiation dose corresponding to the tissue material under the irradiation condition

*FIG. 2*

Obtain an absorbed radiation dose corresponding to each tissue material from an irradiation condition and a conjugate flux corresponding to the tissue material in combination

Select at least one suitable irradiation condition based on the absorbed radiation dose corresponding to each tissue material

*FIG. 3*

Obtain an absorbed radiation dose corresponding to each tissue material from an irradiation condition and a conjugate flux corresponding to the tissue material in combination

Select at least one suitable irradiation condition based on the absorbed radiation dose corresponding to each tissue material

Generate a treatment plan based on the suitable irradiation condition

*FIG.4*

Treatment plan generation apparatus

502

Radiation source module

504

Medical imaging data module

506

Dose evaluation module

508

Irradiation condition selection module

510

Treatment plan generation module

*FIG. 5*

Radiation therapy system

606

Treatment plan generation apparatus

608

Beam control apparatus

604

Beam adjustment apparatus

602

Beam generation apparatus

Irradiated Body

*FIG. 6*

*FIG. 7*

## INTERNATIONAL SEARCH REPORT

| International application No. |
|---|
| **PCT/CN2024/094828** |

| A. | CLASSIFICATION OF SUBJECT MATTER |
|---|---|
| | A61N 5/10(2006.01)i |

According to International Patent Classification (IPC) or to both national classification and IPC

| B. | FIELDS SEARCHED |
|---|---|

Minimum documentation searched (classification system followed by classification symbols)

IPC: A61N

Documentation searched other than minimum documentation to the extent that such documents are included in the fields searched

Electronic data base consulted during the international search (name of data base and, where practicable, search terms used)

CNABS; CNTXT; CNKI; VEN; USTXT; EPTXT; WOTXT: 中硼, 钟万兵, 陈江, 放疗, 反射治疗, 放射疗法, 蒙特卡罗, 蒙特卡洛, 组织, 材料, 信息, 共轭, 通量, 照射, 条件, 吸收, 剂量, 转化因子, 核素, 元素, 密度, 源项, 硼中子, Neuboron therapy, Zhong wanbing, Chen jiang, radiotherapy, Monte Carlo, MC, tissue, adjoint, flux, radiate, absorption, dose, TOPAS, MCNP, PET

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 113797447 A (NEUBORON THERAPY SYSTEM LTD.) 17 December 2021 (2021-12-17) <br> description, paragraphs [0061]-[0102], and figures 1-6 | 1-14 |
| A | CN 115702984 A (HERON NEUTRON MEDICAL CORP.) 17 February 2023 (2023-02-17) <br> entire document | 1-14 |
| A | CN 110570923 A (LINKINGMED TECHNOLOGY CO., LTD.) 13 December 2019 (2019-12-13) <br> entire document | 1-14 |
| A | US 2008004845 A1 (FAILLA GREGORY A. et al.) 03 January 2008 (2008-01-03) <br> entire document | 1-14 |
| A | US 2006259282 A1 (FAILLA GREGORY A. et al.) 16 November 2006 (2006-11-16) <br> entire document | 1-14 |

☑ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **02 September 2024** | **09 September 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/094828** |

| C. | DOCUMENTS CONSIDERED TO BE RELEVANT | |
| --- | --- | --- |
| Category* | Citation of document, with indication, where appropriate, of the relevant passages | Relevant to claim No. |
| A | 毕璇璇等 (BI, Xuanxuan et al.). "基于共轭通量的蒙特卡罗临界硼浓度搜索方法 (Monte Carlo Critical Boron Concentration Search Method Based on Adjoint Flux)" <br> 核技术 (Nuclear Techniques), Vol. 41, No. 7, 31 July 2018 (2018-07-31), <br> entire document | 1-14 |

Form PCT/ISA/210 (second sheet) (July 2022)

# EP 4 717 304 A1

## INTERNATIONAL SEARCH REPORT
### Information on patent family members

International application No.

**PCT/CN2024/094828**

| Patent document cited in search report | | | Publication date (day/month/year) | Patent family member(s) | | | Publication date (day/month/year) |
|---|---|---|---|---|---|---|---|
| CN | 113797447 | A | 17 December 2021 | None | | | |
| CN | 115702984 | A | 17 February 2023 | None | | | |
| CN | 110570923 | A | 13 December 2019 | None | | | |
| US | 2008004845 | A1 | 03 January 2008 | WO | 2008150511 | A2 | 11 December 2008 |
| | | | | WO | 2008150511 | A3 | 22 May 2009 |
| US | 2006259282 | A1 | 16 November 2006 | WO | 2008039215 | A2 | 03 April 2008 |
| | | | | WO | 2008039215 | A3 | 30 April 2009 |

Form PCT/ISA/210 (patent family annex) (July 2022)